# EUROPÄISCHE PATENTSCHRIFT

(11) **EP 0 554 562 B1**
(45) Veröffentlichungstag und Bekanntmachung des Hinweises auf die Patenterteilung: **15.05.1996**
(21) Anmeldenummer: 92121891.3
(22) Anmeldetag: 23.12.1992
(51) Int. Cl.: C01F 7/56

(54) **Verfahren zur Herstellung von Lösungen aus basischen Aluminiumchloriden**
Process for the preparation of basic aluminium chloride solutions
Procédé de préparation de solutions de chlorure d'aluminium basique

(30) Priorität: 01.02.1992 DE 4202937
(43) Veröffentlichungstag der Anmeldung: 11.08.1993
(73) Patentinhaber: A.I.R. Lippewerk Recycling GmbH, D-44536 Lünen (DE)
(72) Erfinder: Kudermann, Gerhard, Dr., W-5305 Alfter (DE); Blaufuss, Karl-Heinz, W-5469 Windhagen (DE); Simbach, Bernd, W-4714 Selm-Bork (DE); Thome, Roland, Dr., W-5300 Bonn 1 (DE); Bings, Hubert, W-4670 Lünen (DE)
(74) Vertreter: Müller-Wolff, Thomas, Dipl.-Ing.

(56) Entgegenhaltungen:
- EP-A- 0 383 736
- WO-A-90/08738
- US-A- 3 909 439
- US-A- 3 953 584
- US-A- 3 957 947
- DATABASE WPIL Week 8610, Derwent Publications Ltd., London, GB; AN 86-064210

## Beschreibung

Die Erfindung betrifft ein Verfahren zur Herstellung von Lösungen aus basischen Aluminiumchloriden mit einem Molverhältnis Al zu Cl von 0,33 bis 0,66 durch Umsetzung eines aluminiumhaltigen Feststoffes mit einer sauren chloridhaltigen, wäßrigen Lösung bei einer Temperatur zwischen 140 und 250 °C und einer Verweilzeit von 2 bis 60 Minuten,
Derartige Polyaluminiumchloridlösungen werden in der Wasserreinigung als Flockungsmittel, als Hydrophobierungsmittel für Textilien, als Keramikbinder, als Antiaspirantien und Deodorantien in der Körperpflege und in der Papierindustrie eingesetzt.

Für die Herstellung von Polyaluminiumchloridlösungen ist eine Reihe verschiedener Verfahren bekannt, wie z. B.:
a) Umsetzung von metallischem Aluminium mit einer substöchiometrischen Menge an Salzsäure. Dieser Weg ist aufgrund der hohen Energiekosten für die Herstellung von Aluminium nicht wirtschaftlich.
b) Lösen von Aluminiumhydroxid in einem Gemisch von Salzsäure und Schwefelsäure bei ca. 120 °C mit nachfolgender Fällung eines Teiles der Sulfationen mit Kalk. Dieses Verfahren führt zu Entsorgungsproblemen des anfallenden Calciumsulfates und zu beschränkten Verwendungsmöglichkeiten der sulfathaltigen Polyaluminiumchloridlösung.
c) Lösen von Aluminiumhydroxid mit einer substöchiometrischen Menge Salzsäure bei ca. 120 °C. Dieser Weg ist sehr zeitaufwendig (ca. 2 Tage).
d) Lösen von Aluminiumhydroxid mit einer substöchiometrischen Menge Salzsäure über 120 °C unter Druck. Dieses Verfahren ist bisher großtechnisch noch nicht gelöst, weil Polyaluminiumchloridlösungen, d. h. Lösungen mit Molverhältnissen von Al/Cl größer 0,33 über 140 °C mit steigender Temperatur instabiler werden und eine chloridhaltige Aluminiumhydroxid-Modifikation ausfällt.

Aus DE-OS 23 09 610 ist ein Verfahren bekannt, bei dem aus einer Suspension von Aluminiumhydroxid in Salzsäure bei ca. 80 °C, unter Einleiten von Chlorwasserstoffgas (HCl) und Halten der Suspension am Siedepunkt (110 °C), Zugabe von weiterem Al(OH)₃ und Erhitzen auf 120 °C für 45 h, eine basische Aluminiumchloridlösung der allgemeinen Zusammensetzung Al₂(OH)₆₋ₙClₙ mit 2<n<6 erhalten wird. Der Nachteil dieses Verfahrens ist der relativ hohe Zeitaufwand und die damit verbundenen Kostenfaktoren wie z.B. Produktivität, Energie und Arbeitszeit.

In DE-OS 25 13 786 ist ein Verfahren beschrieben, bei dem Aluminiumhydroxid mit 30 bis 37 %iger Salzsäure bei 160 bis 270 °C umgesetzt wird. Die Reaktionszeiten liegen dabei zwischen 4 und 45 Stunden. Bei derart hohen Reaktionstemperaturen und langen Verweilzeiten kann die Instabilität der Aluminiumchloridlösung nicht beherrscht werden. Unter den gegebenen Bedingungen ergeben sich dementsprechend Ausbeuteverluste durch Ausfällung einer chloridhaltige Aluminiumhydroxid-Modifikation.

Aus US-A-3 957 947 ist ein Verfahren zur Herstellung von Lösungen aus basischen Aluminiumchloriden durch Umsetzung einer Aufschlämmung von Bauxit, Ton oder Aluminiumhydroxid mit Salzsäure in Rohrreaktoren bekannt. Dabei wird die Umsetzung unter Druck bei einer Temperatur von 120 °C bis 220 °C durchgeführt, wobei die Behandlungszeiten zwischen 2 bis 3 Stunden bei 120 °C und einigen Minuten bei 200 °C schwanken.

Ein Nachteil des bekannten Verfahrens besteht darin, daß bei steigenden Temperaturen die Lösungen instabil werden und es zur Bildung eines chloridhaltigen Aluminiumhydroxid-Niederschlages kommen kann. Dadurch wird ein geringerer Aluminiumgehalt und ein ungünstiges Aluminium/Chlorid-Molverhältnis in der Produktlösung erzielt.

Aufgabe der vorliegenden Erfindung ist es, ein Verfahren zur Herstellung von Lösungen aus basischen Aluminiumchloriden der eingangs genannten Art zu entwickeln, bei dem es gelingt, stabile Produktlösungen auf rationellem Wege mit ausreichend hohen Aluminiumgehalten und Aluminium/Chlorid-Molverhältnissen herzustellen.

Diese Aufgabe wird erfindungsgemäß gelöst durch ein Verfahren mit den Merkmalen gemäß Anspruch 1. Vorteilhafte Ausgestaltungen des Verfahrens ergeben sich aus den Unteransprüchen.

Um die Reaktion von Aluminiumhydroxid mit Salzsäure visuell verfolgen zu können, wurden mit Bezug auf ein Molverhältnis Al zu Cl von 0,33 überstöchiometrische Mengen mit 19 %iger und 38 %iger Salzsäure in dickwandige Pyrexglasrohre eingeschmolzen und auf Temperaturen zwischen 160 °C und 220 °C erhitzt. Die Glasrohre wurden periodisch geschüttelt. Dabei wurde beobachtet, daß sich bei diesen hohen Temperaturen das Aluminiumhydroxid schnell löst und erst nach Erreichen einer klaren Lösung wieder eine Trübung durch einen Niederschlag eintritt. Dieser Niederschlag konnte verhindert werden, wenn die klare Lösung, d.h. nach dem vollständigen Lösen des Aluminiumhydroxids, rasch abgekühlt wurde. Aus diesen Versuchen wurde grundsätzlich gefunden, daß der Löseprozeß von der Bildung des chloridhaltigen Aluminiumhydroxid-Niederschlags bei geeigneter Steuerung der Reaktionsparameter, wie z.B. Verweilzeit und Temperatur, zeitlich getrennt werden kann.

In einer weiteren Versuchsserie wurde untersucht, ob sich mit einer intensiven Rührung die Lösegeschwindigkeit weiter verbessern und eine bessere zeitliche Auflösung des Löseprozesses und der Ausscheidung von chloridhaltigem Aluminiumhydroxid erreichen läßt. Die entsprechenden Versuche erfolgten in einem 1-Liter-Autoklaven mit Magnetrührer und einer säurefesten PTFE-Auskleidung. Hierbei wurde gefunden, daß durch eine Verbesserung der Durchmischung Reaktionszeiten von weniger als 1 Stunde erreicht werden können. Ferner zeigte sich an Hand der Versuche, daß sich mit zunehmender Temperatur und Reaktionszeit innerhalb bestimmter Bereiche die Mengen an gelöstem Aluminium und das Molverhältnis Al/Cl erhöhen lassen. Es wurde weiterhin gefunden, daß die Lösungen mit steigender Temperatur instabiler werden, wodurch bei zu langen Verweilzeiten und gleichzeitig hohen Temperaturen eine chloridhaltige Aluminiumhydroxid-Modifikation ausfällt. Ferner zeigte sich, daß es bei relativ langen Aufheiz- und Abkühlzeiten (hier bedingt durch die PTFE-Auskleidung des Autoklaven) praktisch nicht möglich ist, den Niederschlag der chloridhaltigen Aluminiumhydroxid-Modifikation zu verhindern und eine klare Lösung zu erhalten. Sowohl eine rasche Aufheizung auf die Reaktionstemperatur als auch eine schnelle Abkühlung nach Ende der Verweilzeit wirken sich dementsprechend günstig auf das Verfahren aus.

Nach dem erfindungsgemäßen Verfahren wird eine Suspension aus einem aluminiumhaltigen Feststoff und einer chloridhaltigen, wäßrigen Lösung auf eine Temperatur zwischen 140 und 250 °C aufgeheizt. Bei dieser Temperatur erfolgt die Umsetzung über eine Verweilzeit von 2 bis 60 Minuten. Anschließend wird die Produktlösung abgekühlt. Es entstehen Lösungen aus basischen Aluminiumchloriden mit Al/Cl-Molzahlverhältnissen im Bereich zwischen 0,33 und 0,66, abhängig von der Wahl der Betriebsparameter sowie der Art, Menge und Zusammensetzung der Einsatzstoffe. Das Verhältnis von Feststoff und Lösung in der Ausgangssuspension wird so vorgegeben, daß der Aluminiumgehalt der Produktlösungen vorzugsweise auf Werte oberhalb von ca. 50 g/l eingestellt wird, bei AL/Cl-Molverhältnissen oberhalb von etwa 0,5 (substöchiometrische Zugabe von Cl bezogen auf die Bildung von AlCl₃).

Bei Temperaturen unterhalb von 140 °C ergeben sich unwirtschaftlich lange Reaktionszeiten. Bei Temperaturen oberhalb von 250 °C ist die Einhaltung sehr kurzer Verweilzeiten notwendig, weil in verstärktem Maße eine chloridhaltige Aluminiumhydroxid-Modifikation ausfällt. Dementsprechend wird der Aluminiumgehalt sowie das Al/Cl-Molverhältnis in der Produktlösung verringert. Zur Erzielung von hohen Aluminiumgehalten bei gleichzeitig hohen Molverhältnissen haben sich Reaktionstemperaturen im Bereich zwischen 180 und 220 °C sowie Verweilzeiten im Bereich zwischen 5 und 25 Minuten als besonders günstig erwiesen. Grundsätzlich ist bei höherer Reaktionstemperatur eine geringere Verweilzeit einzustellen und umgekehrt.

Die Herstellung der gewünschten Produktlösungen erfolgt durch Anwendung von Aufheizzeiten bis zu maximal 1 min und eine schnelle Abkühlung der Produktlösung nach Beendigung der Umsetzung. Wird die Produktlösung nach Beendigung der Umsetzungsreaktion innerhalb eines Zeitraumes von maximal 5 min von der Reaktionstemperatur auf eine Temperatur unterhalb von 100 °C abgekühlt, kann die Ausfällung der chloridhaltigen Aluminiumhydroxid-Modifikation deutlich vermindert bzw. vollständig verhindert werden.

Wie bereits dargelegt, lassen sich die Reaktionszeiten deutlich verkürzen durch intensives Vermischen der Ausgangssuspension während der Umsetzung, insbesondere unter turbulenten Strömungsbedingungen.

In bevorzugter Ausführungsform wird das erfindungsgemäße Verfahren kontinuierlich in einem Rohrreaktor durchgeführt. Die optimalen schnellen Aufheiz- und Abkühlzeiten lassen sich in einem Rohrreaktor-Wärmetauscher-System, das 3 bis 4 Wärmetauscher umfaßt, erreichen. Diese sind wärmetechnisch so ausgelegt, daß die durchgesetzte Ausgangssuspension in maximal 1 Minute auf 250 °C aufgeheizt werden kann, z. B. unter Verwendung von Hochdruckdampf, Wärmeträgeröl- oder -salz oder durch elektrische Heizung. An die Wärmetauscher schließt sich eine entsprechend lange Verweilzeitstrecke an, die eine Verweilzeit von 2 bis 60 Minuten gewährleistet. Der Rohrdurchmesser wird so gewählt, daß bei den vorgegebenen Drücken in jedem Falle eine turbulente Strömung erhalten bleibt. Anschließend erfolgt die Kühlung des Reaktionsgemisches entweder durch Entspannung oder Wärmetausch auf eine Temperatur unterhalb von 100 °C innerhalb von 1 bis 5 Minuten.

Der für das erfindungsgemäße Verfahren eingesetzte Feststoff sollte einen Aluminiumgehalt von mindestens 10 Gew.-% aufweisen, da unterhalb dieses Wertes eine wirtschaftliche Verfahrensweise nicht gewährleistet ist. Als Feststoffe eignen sich in besonderer Weise Aluminiumhydroxid, teilweise dehydratisierte Übergangsformen vom Aluminiumhydroxid zum Aluminiumoxid, sowie aluminiumhydroxidhaltige Roh- oder Reststoffe bzw. deren Mischungen. Die verwendeten Feststoffe können dabei auch Eisen enthalten. Dies ist insbesondere dann günstig, wenn die erzeugten Produktlösungen zur Abwasserreinigung verwendet werden sollen. Der bei der Umsetzung in die Produktlösungen überführte Anteil an Eisen begünstig den Einsatz als Flockungsmittel und führt in diesem Anwendungsfall zu verbesserter Filtrierbarkeit und verbessertem Sedimentationsverhalten. Zu den vorteilhaft einsetzbaren Feststoffen zählen insbesondere z. B. Hydrargillit, Gibbsit, Ton oder Bauxit bzw. deren Mischungen.

Für das erfindungsgemäße Verfahren werden vorzugsweise saure chloridhaltige, wäßrige Lösungen verwendet, deren Chlorgehalt mindestens 70 g/l beträgt, entsprechend ca. 2 Mol/l. Das vorgeschlagene Verfahren erlaubt hier den Einsatz von Lösungen unterschiedlichster Art, so daß diese entsprechend dem jeweiligen Verwendungszweck der erzeugten Produktlösungen ausgewählt werden können.

Müssen hohe Anforderungen an die Reinheit der Produkte erfüllt werden, z. B. für kosmetische Zwecke, werden bevorzugt reine Salzsäure- und/oder reine Aluminiumchloridlösungen verwendet. Hier haben sich salzsaure Lösungen mit einem Gehalt an freier Salzsäure im Bereich zwischen 6 und 30 Gew.-% als besonders geeignet erwiesen.

Bei Anwendung der Produkte in Wasserreinigungsverfahren werden bevorzugt Fe-haltige Ausgangslösungen eingesetzt, wie z. B. Eisenchloridlösungen oder Mischungen aus Al- und Fe-haltigen Chloridlösungen. Das erfindungsgemäße Verfahren gestattet zudem die Verwendung und damit eine effektive Verwertung von Restlösungen oder Altsäuren aus industriellen Prozessen, wie z. B. Ätz- oder Beizlösungen, bzw. deren Mischungen.

Weiterhin ist das erfindungsgemäße Verfahren insbesondere auch geeignet, den Aluminiumgehalt sowie das Al/Cl-Molverhältnis von bereits vorliegenden Polyaluminiumchloridlösungen zu erhöhen. In diesem Fall werden als Ausgangslösungen Polyaluminiumchloridlösungen mit einem Aluminiumgehalt unterhalb von 120 g/l bzw. einem Al/Cl-Molverhältnis unterhalb von 0,65 eingesetzt.

Das vorgeschlagene Verfahren erlaubt die rationelle Herstellung von Lösungen aus basischen Aluminiumchloriden mit Al/Cl-Molverhältnissen im Bereich zwischen 0,33 und 0,66. Bei äußerst kurzen Umsetzungszeiten können hohe Aluminiumgehalte ≧ 50 g/l, insbesondere ≧ 100 g/l, und gleichzeitig hohe Al/Cl-Molverhältnisse ≧ 0,5 in den Produktlösungen erzielt werden. Die hohe Produktivität des Verfahrens unter Verringerung bzw. Vermeidung des Ausfallens bereits gelöster Bestandteile führt zu einer Steigerung der Wirtschaftlichkeit. Zudem kann die Umsetzungsreaktion kontinuierlich in einem Rohrreaktor durchgeführt werden. Des weiteren können mit dem erfindungsgemäßen Verfahren u. a. auch Reststoffe mit vergleichsweise geringen Al-Gehalten sowie Rest- bzw. Altlösungen aus industriellen Prozessen nutzbringend verwertet werden.

Nachfolgend wird das erfindungsgemäße Verfahren anhand eines Ausführungsbeispieles näher erläutert.

Die großtechnische Umsetzung des Verfahrens erfolgte in einem Rohrreaktor, der aus einer Vorlage mit Hilfe einer Kolbenmembranpumpe gespeist wird, mit einem Durchsatz von etwa 3 m³/h in mehreren Beispielen. Die bei unterschiedlichen Betriebsparametern (Ausgangsstoffe, Reaktionstemperaturen und Verweilzeiten) erhaltenen Ergebnisse sind in Tabelle 1 zusammengefaßt. Zur Herstellung der Suspension wurde eine salzsaure, aluminiumhaltige Lösung mit Aluminiumhydroxid vermischt.

Die durchgesetzte Supension wurde in dem Rohrreaktor-Wärmetauscher-System innerhalb von max. 1 min bei turbulenten Strömungsbedingungen auf die jeweilige Umsetzungstemperatur aufgeheizt. Die Umsetzung in der Verweilzeitstrecke erfolgte ebenfalls unter turbulenten Strömungsbedingungen. Die Produktlösung wurde nach Beendigung der Umsetzung innerhalb von 1 bis 5 min auf eine Temperatur unterhalb von 100 °C abgekühlt.

Überaschenderweise zeigte sich, daß sich bereits mit einer Verweilzeit von 8 min bei einer maximalen Reaktortemperatur von 200 °C Molverhältnisse von Al/Cl um 0,62 und ein Aluminiumgehalt von 66,4 g/l, entsprechend 5,5 % (Dichte etwa 1,20 g/l), erreichen lassen (Tabelle 1, Beispiel 3). Der Aluminiumgehalt der Produktlösung wird u. a. von den Aluminium- und Chloridgehalten der Einsatzlösung beeinflußt. Bei einer entsprechenden Chargierung und geeigneten Reaktionsbedingungen lassen sich Aluminiumgehalte um 120 g/l, entsprechend etwa 9 %, und einem Molverhältnis (MV) ≧ 0,57 erreichen, was mit Blick auf Transportkosten von weiterem Vorteil ist (Tabelle 1, Beispiele 15 bis 18).

Grundsätzlich können die Produktlösungen rückstandsfrei hergestellt werden. Mögliche Rückstände ergeben sich aus nicht umgesetzten Einsatzstoffen (z.B. Aluminiumhydroxid) sowie aus sekundären Niederschlägen (chloridhaltige Aluminiumhydroxid-Modifikation). Beide Rückstände lassen sich unter den beschriebenen Bedingungen des erfindungsgemäßen Verfahrens weitestgehend vermeiden.

In einem Rohrreaktor lassen sich demnach aus aluminiumhydroxidhaltigen Einsatzstoffen und Salzsäure und/oder aluminiumchloridhaltigen Lösungen auf überraschend rationelle Weise Polyaluminiumchloridlösungen mit relativ hohen Molverhältnissen bei gleichzeitig hohem Aluminiumgehalt herstellen. In der gleichen Verfahrensweise kann im Rohrreaktor auch ein Gemisch aus Aluminiumchlorid-, Eisenchlorid-Lösungen und Aluminiumhydroxid umgesetzt werden. Die auf diese Weise hergestellten Produktlösungen eignen sich in besonderer Weise für die Abwasserreinigung.

Das erfindungsgemäße Verfahren ist daher insbesondere geeignet, große Mengen industrieller salzsaurer Restlösungen kostengünstig zu wertvollen Produkten für die Wasserreinigung sowie zu Ausgangslösungen für weitere technische Anwendungen umzuarbeiten.

**Tabelle 1**

| Technische Versuche im Rohrreaktor | | | | | | | | |
|---|---|---|---|---|---|---|---|---|
| Nr. | Eingangssuspension | | | Reaktionsbedingungen | | Produktlösung | | |
| | Lösung | | Al(OH)₃ g/l | Temp. °C | Verweilzeit min | Al g/l | Cl g/l | MV |
| | Al g/l | Cl g/l | | | | | | |
| 1 | 26 | 170 | 159 | 150 | 6 | 53,8 | 145 | 0,49 |
| 2 | 26 | 170 | 159 | 180 | 8 | 63,5 | 144 | 0,58 |
| 3 | 26 | 170 | 159 | 200 | 8 | 66,4 | 141 | 0,62 |
| 4 | 26 | 170 | 159 | 220 | 8 | 52,6 | 138 | 0,51 |
| 5 | 26 | 170 | 126 | 150 | 18 | 55,0 | 148 | 0,49 |
| 6 | 26 | 170 | 126 | 180 | 18 | 64,1 | 143 | 0,59 |
| 7 | 26 | 170 | 126 | 200 | 18 | 48,1 | 126 | 0,50 |
| 8 | 31 | 200 | 148 | 220 | 18 | 56,7 | 143 | 0,52 |
| 9 | 26 | 170 | 126 | 200 | 24 | 66,4 | 141 | 0,62 |
| 10 | 26 | 170 | 126 | 200 | 24 | 66,0 | 146 | 0,59 |
| 11 | 31 | 200 | 148 | 200 | 24 | 61,6 | 139 | 0,58 |
| 12 | 31 | 200 | 148 | 200 | 24 | 61,4 | 139 | 0,58 |
| 13 | 31 | 200 | 148 | 220 | 16 | 54,8 | 142 | 0,51 |
| 14 | 26 | 170 | 126 | 220 | 24 | 52,6 | 138 | 0,50 |
| 15 | 80 | 300 | 140 | 200 | 24 | 117 | 269 | 0,57 |
| 16 | 80 | 300 | 140 | 200 | 24 | 113 | 260 | 0,57 |
| 17 | 80 | 300 | 150 | 200 | 24 | 120 | 272 | 0,59 |
| 18 | 80 | 300 | 150 | 200 | 24 | 123 | 274 | 0,59 |

## Patentansprüche

1. Verfahren zur Herstellung von Lösungen aus basischen Aluminiumchloriden mit einem Molverhältnis Al zu Cl von 0,33 bis 0,66 durch Umsetzung eines aluminiumhaltigen Feststoffes mit einer sauren chloridhaltigen, wäßrigen Lösung bei einer Temperatur zwischen 140 und 250 °C und einer Verweilzeit von 2 bis 60 Minuten,
dadurch gekennzeichnet,
daß die Suspension in einem Zeitraum von maximal 1 Minute auf die Umsetzungstemperatur aufgeheizt wird und die Produktlösung nach Beendigung der Umsetzung in einem Zeitraum von maximal 5 Minuten von der Umsetzungstemperatur auf eine Temperatur unterhalb 100 °C abgekühlt wird.

2. Verfahren nach Anspruch 1,
dadurch gekennzeichnet,
daß die Umsetzung in einem Rohrreaktor unter turbulenter Strömung durchgeführt wird.

3. Verfahren nach einem der vorhergehenden Ansprüche,
dadurch gekennzeichnet,
daß als Feststoff Aluminiumhydroxid, aluminiumhydroxidhaltige Rohstoffe oder Reststoffe oder deren Mischung verwendet werden.

4. Verfahren nach Anspruch 3,
dadurch gekennzeichnet,
daß der eingesetzte Feststoff Eisen enthält.

5. Verfahren nach Anspruch 3 oder 4,
dadurch gekennzeichnet,
daß als Feststoff Hydrargillit oder Gibbsit verwendet wird.

6. Verfahren nach einem der Ansprüche 1 oder 2,
dadurch gekennzeichnet,
daß eine aluminium- und/oder eisenhaltige Lösung eingesetzt wird.

7. Verfahren nach Anspruch 6,
dadurch gekennzeichnet,
daß Restlösungen oder Altsäuren wie z.B. Ätz- oder Beizlösungen eingesetzt werden.

8. Verfahren nach Anspruch 1 oder 2,
dadurch gekennzeichnet,
daß als Ausgangslösung eine Polyaluminiumchloridlösung mit einem Aluminiumgehalt kleiner als 120 g/l eingesetzt wird.

## Claims

1. A process of producing solutions of alkaline aluminium chlorides with an Al/Cl molar ratio of 0.33 to 0.66 by converting an aluminium containing solid substance with an acid chloride containing aqueous solution at a temperature ranging between 140 and 250 °C and a holding time of 2 to 60 minutes,
characterised in
that the suspension is heated to the conversion temperature in a period of 1 minute maximum and that, after completion of the conversion process, the product solution is cooled from the conversion temperature to a temperature below 100 °C during a period of 5 minutes maximum.

2. A process according to claim 1,
characterised in
that conversion takes place in a tubular reactor under turbulent flow conditions.

3. A process according to any one of the preceding
claims, characterised in
that the solid substance used is aluminium hydroxide, aluminium hydroxide containing raw materials or residual substances or mixtures thereof.

4. A process according to claim 3,
characterised in
that the solid substance used contains iron.

5. A process according to claim 3 or 4,
characterised in
that the solid substance used is hydrargillite or gibbsite.

6. A process according to any one of claims 1 or 2,
characterised in
that an aluminium containing and/or iron containing solution is used.

7. A process according to claim 6,
characterised in
that residual solutions or waste acids such as etching solutions or pickling solutions are used.

8. A process according to claim 1 or 2,
characterised in
that the starting solution is a polyaluminium chloride solution with an aluminium content smaller than 120 g/l.

## Revendications

1. Procédé de préparation de solutions de chlorures d'aluminium basique présentant un rapport molaire de Al à Cl de 0,33 à 0,66, par la réaction d'un solide contenant de l'aluminium avec une solution aqueuse acide contenant du chlorure, à une température comprise entre 140 et 250°C et avec un temps de séjour de 2 à 60 minutes,
procédé caractérisé, en ce que la suspension est chauffée en un espace de temps d'au maximum 1 minute à la température de réaction et, après achèvement de la réaction, la solution de produits est refroidie, en un espace de temps de 5 minutes au maximum, de la température de réaction à une température inférieure à 100°C.

2. Procédé selon la revendication 1, caractérisé en ce que la réaction est conduite dans un réacteur tubulaire parcouru par un courant turbulent.

3. Procédé selon l'une des revendications précédentes, caractérisé en ce qu'on utilise comme solide de l'hydroxyde d'aluminium, des matières premières ou des matières résiduelles contenant de l'hydroxyde d'aluminium ou leurs mélanges.

4. Procédé selon la revendication 3, caractérisé en ce que le solide que l'on utilise contient du fer

5. Procédé selon la revendication 3 ou 4, caractérisé en ce qu'on utilise comme solide de l'hydrargillite ou de la gibbsite.

6. Procédé selon l'une des revendications 1 ou 2, caractérisé en ce qu'on utilise une solution contenant de l'aluminium et/ou du fer.

7. Procédé selon la revendication 6, caractérisé en ce qu'on utilise des solutions résiduaires ou de vieux acides comme par exemple des solutions caustiques, de décapage ou d'attaque ou de mordançage.

8. Procédé selon la revendication 1 ou 2, caractérisé en ce qu'on utilise comme solution de départ une solution de poly(chlorure d'aluminium) ayant une teneur en aluminium inférieure à 120 g/l.
